(19) Europäisches Patentamt
European Patent Office
Office européen des brevets

(11) Veröffentlichungsnummer: **0 002 679**
**B1**

(12) **EUROPÄISCHE PATENTSCHRIFT**

(45) Veröffentlichungstag der Patentschrift: **15.07.81**

(21) Anmeldenummer: **78101536.7**

(22) Anmeldetag: **04.12.78**

(51) Int. Cl.³: **C 07 C 33/50,** C 07 C 33/46, C 07 D 213/30, C 07 D 231/12, C 07 D 307/42, C 07 D 333/16, A 01 N 31/04

(54) **1-Halogen-1-propin-3-ole, Verfahren zu ihrer Herstellung sowie ihre Verwendung als Fungizide.**

(30) Priorität: **15.12.77 DE 2756031**

(43) Veröffentlichungstag der Anmeldung:
**11.07.79 Patentblatt 79/14**

(45) Bekanntmachung des Hinweises auf die Patenterteilung:
**15.07.81 Patentblatt 81/28**

(84) Benannte Vertragsstaaten:
**BE CH DE FR GB IT NL SE**

(56) Entgegenhaltungen:
**CH - A - 601 151**
**FR - A - 913 365**
**FR - A - 1 068 853**
**FR - A - 2 064 390**
**FR - A - 2 314 709**
**FR M 3392**
**FR M 3826**
**GB - A - 970 663**
**GB - A - 1 083 936**
**US - A - 2 989 568**
**US - A - 3 655 359**
**US - A - 3 812 262**

(73) Patentinhaber: **BAYER AG**
**Zentralbereich Patente, Marken und Lizenzen**
**D-5090 Leverkusen 1, Bayerwerk (DE)**

(72) Erfinder: **Jäger, Gerhard, Dr.**
**Claudiusweg 5**
**D-5600 Wuppertal (DE)**
Erfinder: **Klauke, Erich, Dr.**
**Eichendorffweg 8**
**D-5068 Odenthal (DE)**
Erfinder: **Brandes, Wilhelm, Dr.**
**Eichendorffstrasse 3**
**D-5653 Leichlingen (DE)**
Erfinder: **Frohberger, Paul-Ernst, Dr.**
**Willi-Baumeister-Strasse 5**
**D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 679 B1

1-Halogen-1-propin-3-ole, Verfahren zu ihrer Herstellung sowie ihre Verwendung als Fungizide

Die vorliegende Erfindung betrifft neue 1-Halogen-1-propin-3-ole, ein Verfahren zu ihrer Herstellung sowie ihre Verwendung als Fungizide.

Es ist bereits bekannt geworden, daß Azolylalkine, wie beispielsweise 3-Imidazol-1-yl-3-isopropyl-3-phenyl-1-propin, 1,1-Diphenyl-1-imidazol-1-yl-2-octin oder 1-Brom-3,3-diphenyl-3-imidazol-1-yl-1-propin, gute fungizide Eigenschaften aufweisen (vergleiche DT—OS 2 128 700 [LeA 13 716]). Deren Wirksamkeit ist jedoch, insbesondere bei niedrigen Aufwandmengen und -konzentrationen, nicht immer ganz befriedigend. Außerdem sind ihre Pflanzenwertäglichkeit sowie Saatgutverträglichkeit bei der Verwendung als Saatgutbeizmittel nicht immer ganz zufriedenstellend.

Es wurden nun als neue Verbindungen die 1-Halogen-1-propin-3-ole der Formel

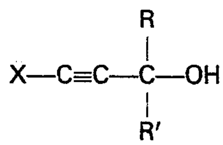

(I)

in welcher

R für Phenyl steht, welches gegebenenfalls einfach oder mehrfach substituiert sein kann durch Halogen, Alkyl mit 1—4 Kohlenstoffatomen, Alkoxy und Alkylthio mit jeweils 1 oder 2 Kohlenstoffatomen, Halogenalkyl und Halogenalkoxy mit jeweils bis zu 2 Kohlenstoff- und bis zu 5 Halogenatomen, ferner durch Amino, Cyano und Nitro, sowie durch Phenyl und Phenoxy, die gegebenenfalls durch Halogen oder Alkyl mit 1 bis 2 Kohlenstoffatomen substituiert sein können, und ferner für Pyridyl, Furyl, Thienyl, oder Pyrazolyl steht, wobei die genannten Reste durch Halogen und Alkyl mit 1 oder 2 Kohlenstoffatomen einfach oder mehrfach substituiert sein können,

R' für Phenyl steht, welches gegebenenfalls die oben beim Rest R für Phenyl aufgezählten Substituenten besitzen kann, und

X für Halogen, steht,

gefunden. Sie weisen starke fungizide Eigenschaften auf.

Weiterhin wurde gefunden, daß man die 1-Halogen-1-propin-3-ole der Formel (I) erhält, wenn man 1-Propin-3-ole der Formel

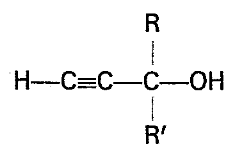

(II)

in welcher

R und R' die oben angegebene Bedeutung haben,

mit Halogenen in Gegenwart einer wässrigen Alkalihydroxid-Lösung und in Gegenwart eines Verdünnungsmittels umsetzt.

Ueberraschenderweise zeigen die erfindungsgemäßen 1-Halogen-1-propin-3-ole eine erheblich höhere fungizide Wirkung als die aus dem Stand der Technik bekannten Azolylalkine, welche chemisch und wirkungsmäßig naheliegende Verbindungen sind. Die erfindungsgemäßen Wirkstoffe stellen somit eine Bereicherung der Technik dar.

Verwendet man 3-Phenyl-3-pyridin-4-yl-1-propin-3-ol und Jod als Ausgangstoffe. so kann der Reaktionsablauf durch das folgende Formelschema wiedergegeben werden:

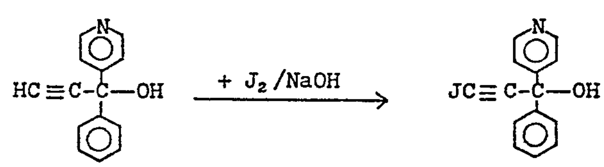


Die als Ausgangstoffe zu verwendenden 1-Propin-3-ole sind durch die Formel (II) allgemein definiert.

Die Verbindungen der Formel (II) sind bekannt oder können auf bekannte Art und Weise hergestellt werden (vergleiche DT—OS 2 438 462 und die dort zitierten Literaturstellen). Man erhält sie z.B. durch Äthinylierung entsprechender Ketone, indem flüssiges Ammoniak auf −60 bis −70°C gekühlt und Acetylen eingeleitet wird, wobei dem flüssigen Ammoniak in mehreren kleinen Portionen metallisches Natrium oder Kalium zugesetzt wird; danach läßt man langsam das zu äthinylierende Keton, gegebenenfalls in Gegenwart eines Lösungsmittels, zu diesem heterogenen Reaktionsgemisch

zufließen. Nach Ablauf der Reaktion läßt man das flüssige Ammoniak über Nacht verdunsten und isoliert die Endprodukte in üblicher Weise. Die Aethinylierung kann auch durchgeführt werden, indem man das entsprechende Keton in Gegenwart eines Alkalimetall-Derivats eines tertiären Alkanols in einem aprotischen organischen Lösungsmittel mit Acetylen umsetzt (vgl. auch Herstellungsbeispiele).

Als Beispiele für die erfindungsgemäß als Ausgangsstoffe zu verwendenden 1-Propin-3-ole der Formel (II) seien beispielsweise die folgenden Verbindungen der Tabelle 1 genannt:

TABELLE 1

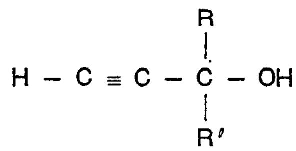

| R | R' | Schmelzpunkt (°C) bzw. Siedepunkt (°C) /mmHg |
|---|---|---|
| 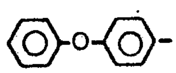 | 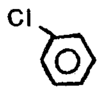 | zähfl.Oel |
| 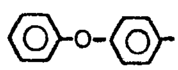 | 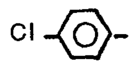 | 86–87 |
| 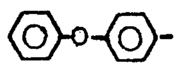 | 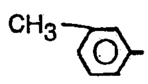 | 78–79 |
| 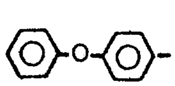 | 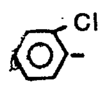 | zähfl.Oel |
| 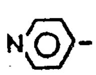 | 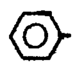 | 165–166 |
| 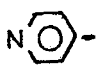 | 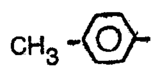 | 191–93 |
| 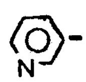 | 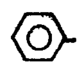 | 140–41 |
| 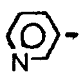 | 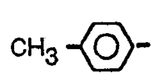 | 144–45 |
| 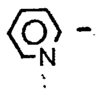 | 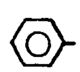 | 65–66 |
| 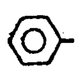 | 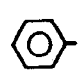 | 48,5 |
| 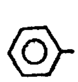 | 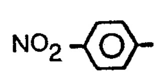 | 87–88 |

TABELLE 1 (Fortsetzung)

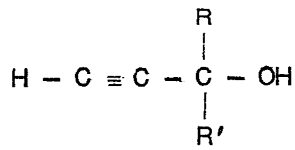

| R | R' | Schmelzpunkt (°C) bzw Siedepunkt (°C) /mmHg |
|---|---|---|
| 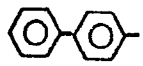 | 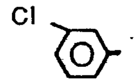 | 123–24 |
| 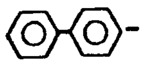 | 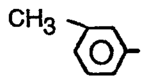 | 104–06 |
| 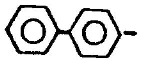 | 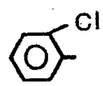 | 126–28 |
| 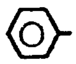 | 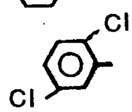 | 85–86 |
| 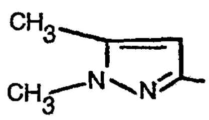 | 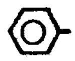 | 111–12 |
| 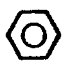 | 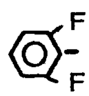 | 126 /0,1 |
| 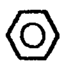 | 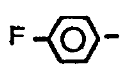 | 118–22 /0,1 |
| 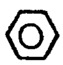 | 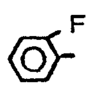 | 118–21 /0,1 |
| 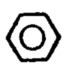 | 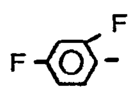 | 140–45 /0,05 |
| 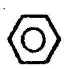 | 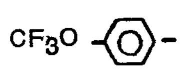 | 130/0,05 |
| 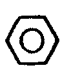 | 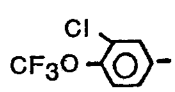 | 125–30 /0,07 |
| 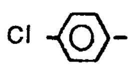 | 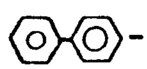 | 106–07 |
| 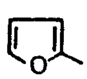 | 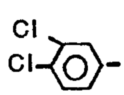 | 146–48 /0,08 |
| 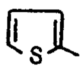 | 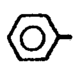 | 130–36 /0,15 |

Als Halogene werden für die erfindungsgemäße Reaktion vorzugsweise Brom oder Jod verwendet.

Für die erfindungsgemäße Umsetzung kommen als Verdünnungsmittel vorzugsweise protische Lösungsmittel in Frage. Hierzu gehören vorzugsweise Alkohole, wie Methanol, Aethanol, Propanol, Isopropanol und Glykolmonomethyläther. Gegebenenfalls kann auch in Mischungen derselben mit anderen Solventien gearbeitet werden, wie z.B. mit Wasser oder mit organischen Aminen, wie Pyridin, Chinolin oder Picolinen.

Die erfindungsgemäße Umsetzung wird in Gegenwart von Alkalihydroxid durchgeführt. Hierzu verwendet man vorzugsweise wässrige Lösungen von Natrium- oder Kaliumhydroxid.

Die Reaktionstemperaturen können beim erfindungsgemäßen Verfahren in einem größeren Bereich variiert werden. Im allgemeinen arbeitet man zwischen 0 und 50°C, vorzugsweise zwischen 20 und 30°C.

Bei der Durchführung des erfindungsgemäßen Verfahrens werden entweder das Halogen und die wässrige Alkalihydroxid-Lösung gleichzeitig zur Verbindung der Formel (II) gegeben oder diese wird sofort mit einem Gemisch aus Alkalihydroxid-Lösung und Halogen versetzt. Dabei wird vorzugsweise mit molaren Mengen gearbeitet. Zur Isolierung der Verbindungen der Formel (I) wird das Reaktionsgemisch mit Wasser versetzt und in üblicher Weise aufgearbeitet.

Als besonders wirksame Verbindungen der Formel (I) seien außer den Herstellungsbeispielen und den Beispielen der Tabelle 2 folgende genannt:

1-Jod-3-phenyl-3-pyridin-3-yl-1-propin-3-ol
1-Brom-3-(4-chlorphenyl)-3-pyridin-4-yl-1-propin-3-ol
3-(4-Chlorphenyl)-1-jod-3-pyridin-4-yl-1-propin-3-ol
1-Brom-3-(2,4-dichlorphenyl)-3-pyridin-4-yl-1-propin-3-ol
3-(2,4-Dichlorphenyl)-1-jod-3-pyridin-4-yl-1-propin-3-ol
1-Brom-3-(4-fluorphenyl)-3-pyridin-4-yl-1-propin-3-ol
3-(4-Fluorphenyl)-1-jod-3-pyridin-4-yl-1-propin-3-ol
3,3-Bis-(4-chlorphenyl)-1-brom-1-propin-3-ol
3,3-Bis-(4-chlorphenyl)-1-jod-1-propin-3-ol
1-Brom-3-(4-chlorphenyl)-3-(2,4-dichlorphenyl)-1-propin-3-ol
3-(Chlorphenyl-3-(2,4-dichlorphenyl)-1-jod-1-propin-3-ol
1-Brom-3-(2-chlorphenyl)-3-(4-chlorphenyl)-1-propin-3-ol
3-(2-Chlorphenyl)-3-(4-chlorphenyl)-1-jod-1-propin-3-ol
1-Brom-3-(2-chlorphenyl)-3-(2,4-dichlorphenyl)-1-propin-3-ol
3-(2-Chlorphenyl)-3-(2,4-dichlorphenyl)-1-jod-1-propin-3-ol
1-Brom-3-(3-chlorphenyl-3-(2,4-dichlorphenyl)-1-propin-3-ol
3-(3-Chlorphenyl)-3-(2,4-dichlorphenyl)-1-jod-1-propin-3-ol
1-Brom-3-(3-chlorphenyl)-3-(4-chlorphenyl)-1-propin-3-ol
3-(3-Chlorphenyl)-3-(4-chlorphenyl)-1-jod-1-propin-3-ol
1-Brom-3-(4-bromphenyl)-3-(4-chlorphenyl)-1-propin-3-ol
3-(4-Bromphenyl)-3-(4-chlorphenyl)-1-jod-1-propin-3-ol
1-Brom-3-(4-bromphenyl-3-(2-chlorphenyl)-1-propin-3-ol
3-(4-Bromphenyl)-3-(2-chlorphenyl)-1-jod-1-propin-3-ol

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanze vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von Phytophthora infestans (Braunfäule der Tomate) sowie Venturia-Arten, wie gegen den Erreger des Apfelschorfs (Fusicladium dendriticum), verwendet werden; außerdem auch zur Bekämpfung von Getreidkrankheiten, wie Getreiderost und Weizensteinbrand. Gute Wirkungen werden ebenfalls bei der Bekämpfung von Pyricularia oryzae am Reis erzielt.

Als Pflanzenschutzmittel können die erfindungsgemäßen Wirkstoffe zur Saatgut- oder Bodenbehandlung und zur Behundlung oberirdischer Pflanzenteile benutztwerden.

Die Wirkstoffe, können in die üblichen Formulierungen übergeführt werden, wie Lösungen, Emulsionen, Spritzpulver, Suspension, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haltmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen Pulver, Pasten und Granulate angewendet werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentration in den Anwendungsformen in einem größeren Bereich variiert werden. Sie liegen im allgemeinen zwischen 0,1 und 0,00001 Gewichtsprozent. Vorzugsweise zwischen 0,05 und 0,0001%.

Bei der Saatgutbehandlung werden im allgemeinen Wirkstoffmengen von 0,001 bis 50 g je Kilogramm Saatgut, vorzugsweise 0,01 bis 10 g benötigt.

In entsprechenden Aufwandkonzentrationen zeigen die erfindungsgemäßen Wirkstoffe auch eine akarizide Wirking.

Die vielseitigen Verwendungsmöglichkeiten gehen aus den nachfolgenden Beispielen hervor.

## Beispiel A

Phytophthora-Test (Tomaten)/Protektiv

Lösungsmittel: 4,7 Gewichtsteile Aceton

Emulgator: 0,3 Gewichtsteile Alkyl-aryl-polyglykoläther

Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70% im Gewächschaus. Anschließend werden die Tomatenpflanzen mit einer wässrigen Sporensuspension von Phytophthora infestans inokuliert. Die Pflanzen werden in eine Feuchtkammer mit einer 100%igen

Luftfeuchtigkeit und einer Temperatur von 18 bis 20°C gebracht.

Nach 5 Tagen wird der Befall der Tomatenpflanzen bestimmt. Die erhaltenen Boniturwerte werden auf Prozent Befall umgerechnet. 0% bedeutet keinen Befall, 100% bedeutet, daß die Pflanzen vollständig befallen sind.

Wirkstoff, Wirkstoffkonzentrationen und Ergebnisse werden ermittelt.

In diesem Test zeigen z.B. folgende Verbindungen eine sehr gute Wirkung, die derjenigen der aus dem Stand der Technik bekannten Verbindungen deutlich überlegen ist:

Verbindungen gemäß Herstellungsbeispielen 6, 12, 13, 21, 23, 25, 27, 29 und 31.

Beispiel B

Fusicladium-Test (Apfel)/Protektiv

| | | |
|---|---|---|
| Lösungsmittel: | 4,7 Gewichtsteile | Aceton |
| Emulgator: | 0,3 Gewichtsteile | Alkyl-aryl-polyglykoläther |
| Wasser: | 95,0 Gewichtsteile | |

Man vermischt die für die gewünschte Wirkstoffkonzentration in der Spritzflüssigkeit nötigen Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächschaus. Anschließend werden sie mit einer wässrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. In diesem Test zeigen z.B, folgende Verbindungen eine sehr gute Wirking, die derjenigen der aus dem Stand der Technik bekannten Verbindungen deutlich überlegen ist:

Verbindungen gemäß Herstellungsbeispielen 2, 8, 12, 13, 23, 25, 27 und 29.

Beispiel C

Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Alkyl-aryl-polyglykoläther auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasserager. Nach Antrocknen der Sporensuspension besprüht man die Weizenpflanzen mit der Wirkstoffzubereitung taufeucht und stellt sie zur Inkubation für 24 Stunden bei etwa 20°C und einer 100%igen Luftfeuchtigkeit in ein Gewächshaus.

Nach 10 Tagen Verweilzeit der Pflanzen bei einer Temperatur von 20°C und einer Luftfeuchtigkeit von 80—90% wertet man den Besatz der Pflanzen mit Rostpusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Rostbefall ist.

Wirkstoffe, Wirkstoffkonzentrationen in der Spritzbrühe und Befallsgrade werden ermittelt. In diesem Test zeigen z.B. folgende Verbindungen eine sehr gute Wirkung, die derjenigen der aus dem Stand der Technik bekannten Verbindungen deutlich überlegen ist:

Verbindungen gemäß Herstellungsbeispielen 12, 13, 23, 25, 27 und 31.

Beispiel D

Saatgutbeizmittel-Test/Weizensteinbrand (samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Man kontaminiert Weizensaatgut mit 5g Chlamydosporen von Tilletia caries pro kg Saatgut. Zur Beizung schüttelt man das Saatgut mit dem Beizmittel in einer verschlossenen Glasflasche. Das Saatgut wird auf feuchtem Lehm unter einer Deckschicht aus einer Lage Mull und 2 cm mäßig feuchter

Komposterde 10 Tage lang im Kühlschrank bei 10°C optimalen Keimungsbedingungen für die Sporen ausgesetzt.

Anschließend bestimmt man mikroskopisch die Keimung der Sporen auf dem Weizenkörnern, die jeweils mit rund 100 000 Sporen besetzt sind. Der Wirkstoff ist umso wirksamer je weniger Sporen gekeimt sind.

Wirkstoffe, Wirkstoffkonzentrationen im Beizmittel, Beizmittelaufwandmengen und Keimprozente der Sporen werden ermittelt. In diesem Test zeigen z.B. folgende Verbindungen eine sehr gute Wirkung, die derjenigen der aus dem Stand der Technik bekannten Verbindungen deutlich überlegen ist: Verbindungen gemäß Herstellungsbeispielen 1, 12 und 13.

*Herstellungsbeispiele*

Beispiel 1

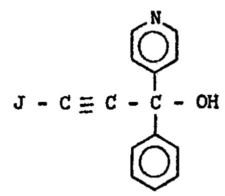

Zu einer Suspension von 42g (0,2 Mol) 3-Phenyl-3-pyridin-4-yl-1-propin-3-ol in 500 ml Methanol werden bei Raumtemperatur unter gleichzeitigem Zutropfen von 80ml konzentrierter Natronlauge 50,8g (0,2 Mol) Jod allmählich eingetragen. Man erhält dabei eine nahezu klare Lösung. Nach dreistündigem Rühren bei Raumtemperatur wird mit viel Wasser verdünnt, die ausgeschiedenen Kristalle werden abgesaugt, mit Wasser gewaschen und im Vakuum getrocknet. Nach Umkristallisation aus Methanol erhält man 53g (79,2% der Theorie) 1-Jod-3-phenyl-3-pyridin-4-yl-1-propin-3-ol als farblose Kristalle vom Schmelzpunkt 175°C (Zers.).

*Herstellung des Vorproduktes*

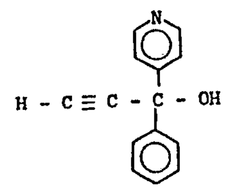

In eine Suspension von 174g (1,55 Mol) Kalium-tert.-butylat in 750 ml Tetrahydrofuran wird ca. 30 Minuten lang Acetylen eingeleitet. Zu diesem Reaktionsgemisch werden 198g (1,08 Mol) 4-Benzoylpyridin in 400ml Tetrahydrofuran zugetropft, wobei gleichzeitig weiter ca. 1 Stunde lang Acetylen eingeleitet wird. Man läßt 30 Minuten nachrühren, versetzt mit verdünnter Salzsäure, saugt das ausgefallene Salz ab und trennt die organische Phase ab. Diese wird im Vakuum eingeengt. Der feste Rückstand wird mit Wasser verrieben, abgesaugt und mit Methanol-Wasser (1:1) gewaschen. Man erhält 170g (75% der Theorie) 3-Phenyl-3-pyridin-4-yl-1-propin-3-ol vom Schmelzpunkt 165—66°C.

Beispiel 2

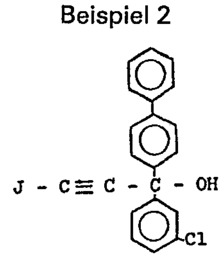

Zu einer Lösung von 31,9g (0,1 Mol) 3-(4-Biphenylyl)-3-(3-chlorphenyl)-1-propin-3-ol in 350 ml Methanol werden bei 20°C unter leichter Außenkühlung gleichzeitig 40 ml konzentrierte Natronlauge und 25,4 g (0,1 Mol) Jod unter Rühren eingetragen. Man läßt drei Stunden rühren, filtriert von einer geringen Trübung ab und rührt das Filtrat in 1000 ml Wasser ein. Dabei scheidet sich das Reaktionsprodukt als halbfeste Masse ab. Man dekantiert die überstehende Flüssigkeit ab, nimmt den Rückstand in Essigester auf, wäscht mehrmals mit 100 ml Wasser und trocknet über Natriumsulfat. Danach engt man durch Abdestillieren des Lösungsmittels im Vakuum ein und löst den öligen Rückstand in wenig Aether. Nach Zugabe von Petroläther kommt es zur Kristallisation. Man erhält 25,3 g (56,9% der Theorie) 3-(4-Biphenylyl)-3-(3-chlorphenyl)-1-jod-1-propin-3-ol als farblose Kristalle vom Schmelzpunkt 99—100°C.

Beispiel 3

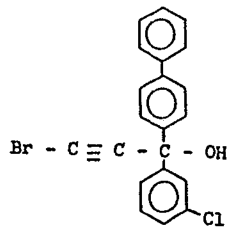

Zu 129 ml (0,32 Mol) 10%-iger Natronlauge werden bei 0 bis 5°C 8,2 ml (0,16 Mol) Brom eingerührt. Zu dieser Lösung läßt man bei Raumtemperatur 44,6 g (0,14 Mol)3-(4-Biphenylyl)-3-(3-chlorphenyl)-1-propin-3-ol in 150 ml Pyridin zutropfen, wobei die Temperatur des Reaktionsgemisches auf 30°C ansteigt. Man läßt 24 Stunden bei Raumtemperatur stehen, filtriert die ausgeschiedenen Kristalle ab und wäscht gut mit Wasser nach. Nach dem Trocknen und Umkristallisieren aus Petroläther/Aether erhält man 43g (77,2% der Theorie) 1-Brom-3-(4-biphenylyl)-3-(3-chlorphenyl)-1-propin-3-ol vom Schmelzpunkt 106—107°C.

In analoger Weise werden die Verbindungen der folgenden Tabelle 2 erhalten.

TABELLE 2

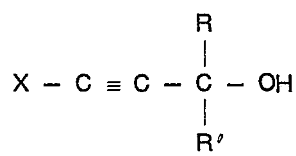

| Beispiel Nr. | R | R′ | X | Schmelzpunkt (°C) bzw. Brechungsindex |
|---|---|---|---|---|
| 4 | 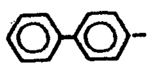 | 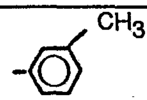 | J | 137–39 |
| 5 | 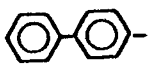 | 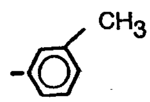 | Br | 131–32 |
| 6 | 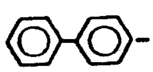 | 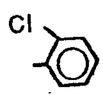 | J | zahfl.Oel |
| 7 | 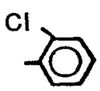 | 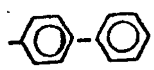 | Br | 77–79 (xPyridin) |
| 8 | 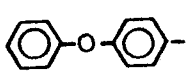 | 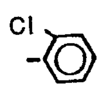 | J | 104–05 |
| 9 | 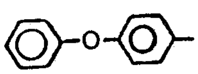 | 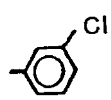 | J | zähfl.Oel |
| 10 | 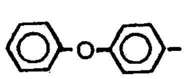 | 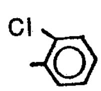 | Br | 95–96 |
| 11 | 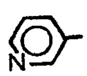 | 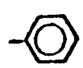 | Br | 107–08 |

TABELLE 2 (Fortsetzung)

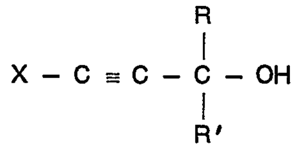

| Beispiel Nr. | R | R' | X | Schmelzpunkt (°C) bzw. Brechungsindex |
|---|---|---|---|---|
| 12 | 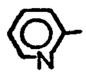 | 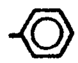 | J | 86–87 |
| 13 | 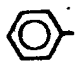 | 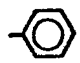 | J | 106–07 |
| 14 | 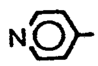 | 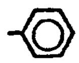 | Br | 166–67 |
| 15 | 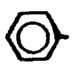 | 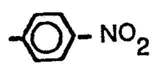 | J | 116–18 |
| 16 | 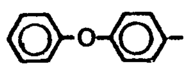 | 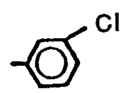 | Br | zähfl.Oel |
| 17 | 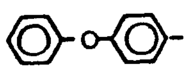 | 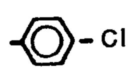 | J | zähfl.Oel |
| 18 | 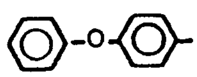 | 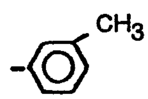 | J | 82–83 |
| 19 | 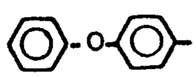 | 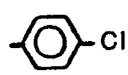 | Br | zähfl.Oel |
| 20 | 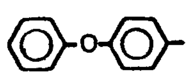 | 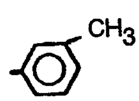 | Br | zähfl.Oel |
| 21 | 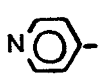 | 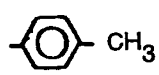 | J | 179–80 (Zers.) |
| 22 | 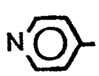 | 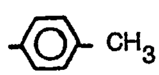 | Br | 189 (Zers.) |
| 23 | 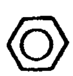 | 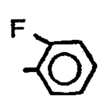 | J | 81–82 |
| 24 | 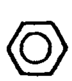 | 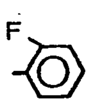 | Br | $n_D^{20}$ : 1,6040 |

TABELLE 2 (Fortsetzung)

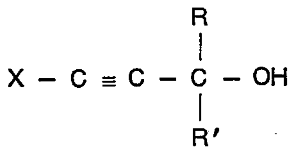

| Beispiel Nr. | R | R' | X | Schmelzpunkt (°C) bzw. Brechungsindex |
|---|---|---|---|---|
| 25 | 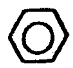 | 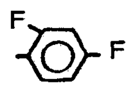 | J | $n_D^{20}$ : 1,6098 |
| 26 | 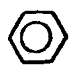 | 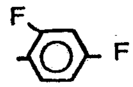 | Br | $n_D^{20}$ : 1,5845 |
| 27 | 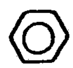 | 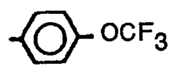 | J | $n_D^{20}$ : 1,5697 |
| 28 | 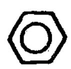 | 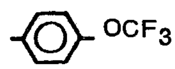 | Br | $n_D^{20}$ : 1,5497 |
| 29 | 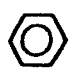 | 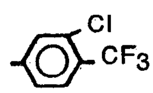 | J | $n_D^{20}$ : 1,5860 |
| 30 | 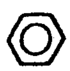 | 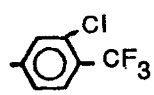 | Br | $n_D^{20}$ : 1,5683 |
| 31 | 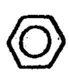 | 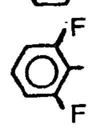 | J | 93–95 |
| 32 | 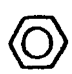 | 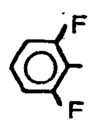 | Br | 60–61 |
| 33 | 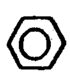 | 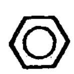 | Br | 60–62 |
| 34 | 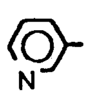 | 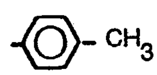 | J | 148–50 |
| 35 | 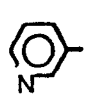 | 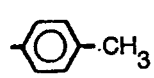 | Br | 152 (Zers.) |

**Patentansprüche**

1. 1-Halogen-1-propin-3-ole der Formel

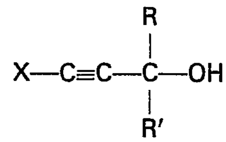

(I)

in welcher

R für Phenyl steht, welches einfach oder mehrfach substituiert sein kann durch Halogen, Alkyl mit 1—4 Kohlenstoffatomen, Alkoxy und Alkylthio mit jeweils 1 oder 2 Kohlenstoffatomen, Halogenalkyl und Halogenalkoxy mit jeweils bis zu 2 Kohlenstoff- und bis zu 5 Halogenatomen, ferner durch Amino, Cyano und Nitro, sowie durch Phenyl und Phenoxy, die durch Halogen oder Alkyl mit 1 bis 2 Kohlenstoffatomen substituiert sein können, und ferner für Pyridyl, Furyl, Thienyl, oder Pyrazolyl steht, wobei die genannten Reste durch Halogen und Alkyl mit 1 oder 2 Kohlenstoffatomen einfach oder mehrfach substituiert sein können,

R' für Phenyl steht, welches die oben beim Rest R für Phenyl aufgezählten Substituenten besitzen kann, und

X für Halogen, steht.

2. Verfahren zur Herstellung von 1-Halogen-1-propin-3-olen, dadurch gekennzeichnet, daß man 1-Propin-3-ole der Formel

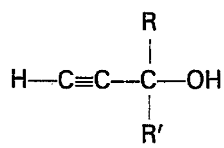

(II)

in welcher

R und R' die in Anspruch 1 angegebene Bedeutung haben, mit Halogenen in Gegenwart einer wässrigen Alkalihydroxid-Lösung und in Gegenwart eines Verdünnungsmittels umsetzt.

3. Fungizide Mittel, gekennzeichnet durch einen Gehalt an mindestens einem 1-Halogen-1-propin-3-ol gemäß Anspruch 1.

4. Verwendung von 1-Halogen-1-propin-3-olen gemäß Anspruch 1 zur Bekämpfung von Pilzen.

5. Verfahren zur Herstellung von fungiziden Mitteln, dadurch gekennzeichnet, daß an 1-Halogen-1-propin-3-ole gemäß Anspruch 1 mit Streckmitteln und/oder oberflächenaktiven Mitteln vermischt.

**Claims**

1. 1-Halogeno-1-propin-3-ols of the formula

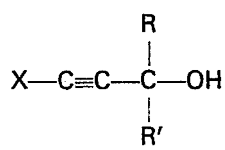

(I)

in which

R represents phenyl which can be monosubstituted or polysubstituted by halogen, alkyl with 1—4 carbon atoms, alkoxy and alkylthio with in each case 1 or 2 carbon atoms, halogenoalkyl and halogenalkoxy with in each case up to 2 carbon atoms and up to 5 halogen atoms, and furthermore by amino, cyano and nitro, and by phenyl and phenoxy which can be substituted by halogen or alkyl with 1 to 2 carbon atoms, and furthermore represents pyridyl, furyl, thienyl or pyrazolyl, it being possible for the named radicals to be monosubstituted or polysubstituted by halogen and alkyl with 1 or 2 carbon atoms,

R' represents phenyl which can have the substituents listed above for phenyl in the case of the radical R, and

X represents halogen.

2. Process for the preparation of 1-halogeno-1-propin-3-ols, characterised in that 1-propin-3-ols of the formula

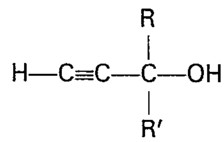

(II)

in which

R and R′ have the meaning indicated above, are reacted with halogens in the presence of an aqueous alkali metal hydroxide solution and in the presence of a diluent.

3. Fungicidal agents, characterised in that they contain at least one 1-halogeno-1-propin-3-ol according to Claim 1.

4. Use of 1-halogeno-1-propin-3-ols according to Claim 1 for combating fungi.

5. Process for the preparation of fungicidal agents, characterised in that 1-halogeno-1-propin-3-ols according to Claim 1 are mixed with extenders and/or surface-active agents.

**Revendications**

1. 1-halogéno-1-propyne-3-ols de formule

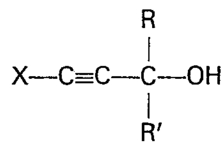

(I)

dans laquelle

R représente un groupe phényle qui peut être mono- ou poly-substitué par des halogènes, des groupes alkyle en $C_1$—$C_4$, alcoxy et alkylthio contenant chacun 1 ou 2 atomes de carbone, halogénoalkyle et halogénoalcoxy contenant chacun jusqu'à 2 atomes de carbone et jusqu'à 5 atomes d'halogènes, ou encore par des groupes amino, cyano et nitro, ou encore par des groupes phényle et phénoxy qui peuvent être substitués par des halogènes ou des groupes alkyle en $C_1$—$C_2$, ou encore un groupe pyridyle, furyle, thiényle ou pyrazolyle, les restes mentionnés pouvant être mono- ou poly-substitués par des halogènes et des groupes alkyle en $C_1$—$C_2$,

R′ représente un groupe phényle qui peut porter les substituants énumérés pour le groupe phényle en référence au symbole R, et

X représente un halogène.

2. Procédé de préparation de 1-halogéno-1-propyne-3-ols, caractérisé en ce que l'on fait réagir des 1-propyne-3-ols de formule

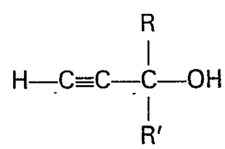

(II)

dans laquelle R et R′ ont les significations indiquées dans la revendication 1, avec des halogènes en présence d'une solution aqueuse d'hydroxyde alcalin et en présence d'un diluant.

3. Produit fongicide caractérisé en ce qu'il contient au moins un 1-halogéno-1-propyne-3-ol selon la revendication 1.

4. Utilisation des 1-halogéno-1-propyne-3-ols selon la revendication 1 dans la lutte contre les mycètes.

5. Procédé de préparation de produits fongicides, caractérisé en ce que l'on mélange des 1-halogéno-1-propyne-3-ols selon la revendication 1 avec des diluants et/ou des agents tensioactifs.